(19) Europäisches Patentamt
European Patent Office
Office européen des brevets

(11) Veröffentlichungsnummer: **0 079 005**
**B1**

(12) # EUROPÄISCHE PATENTSCHRIFT

(45) Veröffentlichungstag der Patentschrift: **26.02.86**

(21) Anmeldenummer: **82109998.3**

(22) Anmeldetag: **29.10.82**

(51) Int. Cl.⁴: **C 07 C 51/56**, C 07 C 63/06, C 07 C 61/08, C 07 C 63/70, C 07 C 53/12, C 07 C 53/122, C 07 C 53/128, C 07 C 79/46, C 07 D 307/54, C 07 C 65/21

(54) **Verfahren zur Herstellung von Carbonsäureanhydriden.**

(30) Priorität: **11.11.81 DE 3144791**

(43) Veröffentlichungstag der Anmeldung: **18.05.83 Patentblatt 83/20**

(45) Bekanntmachung des Hinweises auf die Patenterteilung: **26.02.86 Patentblatt 86/9**

(84) Benannte Vertragsstaaten: **AT BE CH DE FR GB IT LI NL**

(56) Entgegenhaltungen:
**DE - C - 171 146**
**DE - C - 394 730**

**JOURNAL OF THE CHEMICAL SOCIETY, PERKIN TRANSACTIONS 1, Nr. 5, 1976, Seiten 564-569, M. MIKOLAJCZYK et al.: "Organosulphur compounds. Part VIII. Reaction of monothiocarboxylic acids with dicyclohexylcarbodi-imide and other reactions leading to monothio-anhydrides"**
**CHEMICAL ABSTRACTS, Band 62, Nr. 3, 1. Februar 1965, Spalte 2700e, Columbus, Ohio, USA S.H. DANDEGAONKER: "Reactions of silicon tetrachloride with carboxylic acids, anhydrides, and salts"**

(73) Patentinhaber: **BAYER AG, Konzernverwaltung RP Patentabteilung, D-5090 Leverkusen 1 Bayerwerk (DE)**

(72) Erfinder: **Findeisen, Kurt, Dr., In der Follmühle 10, D-5068 Odenthal 2 (DE)**

EP 0 079 005 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Die vorliegende Erfindung betrifft ein chemisch eigenartiges Verfahren zur Herstellung von Carbonsäureanhydriden aus den Trimethylsilylestern der entsprechenden Carbonsäuren.

In der Literatur sind bereits zahlreiche Methoden zur Synthese von Carbonsäurenhydriden beschrieben worden (vgl. z.B. Houben-Weyl, Methoden der Organischen Chemie, 4. Auflage, Band VIII, S. 476—480 (1952)). Es ist bisher aber kein Verfahren bekannt geworden, das es erlaubt, die Trimethylsilylester der Carbonsäuren direkt in die entsprechenden Anhydride zu überführen.

Aus der Literatur ist ferner ein Verfahren zur Herstellung von Thiolcarbonsäureanhydriden durch Umsetzung von Thioncarbonsäure-O-trimethylsilylestern mit Carbonsäurechloriden bekannt (vgl. M. Mikolajczyk et al., Journal of the Chemical Society, Perkin Transactions I, Nr. 5 (1976), Seiten 564—569, insbesondere Seiten 565, 567 und 569). Läßt man beispielsweise eine Mischung von Thionessigsäure-O-trimethylsilylester ($CH_3$—CS—$OSi(CH_3)_3$) und überschüssigem Acetylchlorid ($CH_3$—CO—Cl) bei Raumtemperatur über Nacht stehen, so erhält man nach Abdestillieren des gebildeten Trimethylsilylchlorids und des überschüssigen Acetylchlorids in hoher Ausbeute Diacetylsulfid ($CH_3$—CO—S—CO—$CH_3$). Bei dieser bekannten Reaktion handelt es sich um eine Umlagerung: Der Thionester (A) wird unter Umlagerung umgesetzt zu einem Thiolanhyhdrid (B), das auch als Diacyl-sulfid bezeichnet werden kann;

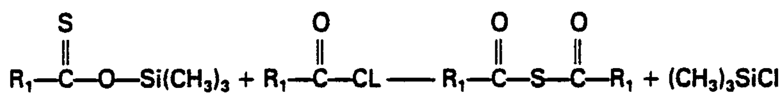

(A) (B)

$R_1$ = —$CH_3$, —$C_2H_5$, —$C(CH_3)_3$, —$C_6H_5$, —$C_6H_4$—$CH_3$(o), —$C_6H_4$—$NO_2$(p).

Das Vorhandensein des Schwefels im Ausgangsprodukt (A) war daher als essentiell für die Reaktion anzusehen. Dies gilt insbesondere deshalb, weil verschiedene Reaktionen von Thioestern bekannt sind, welche mit den analogen Carbonsäureestern nicht ablaufen.

Als Beispiel sei die Umstezung des Thionameisensäureethylesters (C) mit Kaliumcyanamid zu Kalium—N,N'—di-cyan-formamidin (D) genannt (vgl. Archiv der Pharmazie *303,* 632 (1970)):

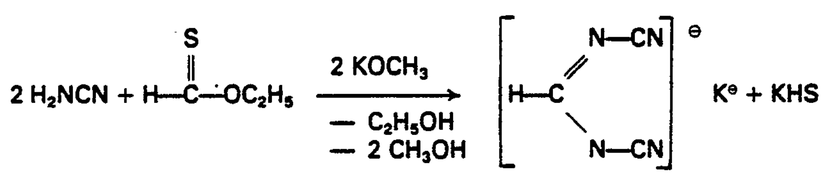


(C) (D)

Wird diese Reaktion mit Ameisensäureethylester (E) an Stelle des Thionameisensäureethylesters (C) durchgeführt, so wird nur Kalium-formylcyanamid (F) erhalten:

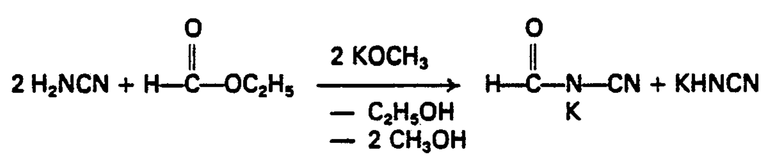


(E) (F)

Es wurde nun gefunden, daß man Carbonsäureanhydride der allgemeinen Formel (I)

$$R—CO—O—CO—R' \qquad (I)$$

in welcher

R und R' gleich oder verschieden sein Können und für gegebenenfalls substituiertes Alkyl mit 1 bis 18 Kohlenstoffatomen, gegebenefalls, substituiertes Cycloalkyl mit 3 bis 12 Kohlenstoffatomen, gegebenefalls substituiertes Aryl oder für einen gegebenenfalls substituierten 5-oder 6-gliedrigen heterocyclischen Rest stehen, der zusätzlich noch mit einem Benzolring annelliert sein kann,

mit sehr hoher Ausbeute und Reinheit erhält, wenn man Carbonsäure-trimethylsilylester der allgemeinen Formel (II)

$$R—CO—OSi(CH_3)_3 \qquad (II)$$

in welcher

R die oben angegebene Bedeutung hat,

mit Carbonsäurehalogeniden der allgemeinen Formel (III)

$$R'—CO—X \qquad (III)$$

in welcher

R' die oben angegebene Bedeutung hat und

X für Halogen steht,

gegebenenfalls in Gegenwart eines Katalysators und gegebenenfalls in Gegenwart eines Verdünnungsmittels bei Temperaturen zwischen 20°C und 250°C umsetzt und das im Reaktionsverlauf in äuqivalenter Menge entstehende Trimethylsilylhalogenid der Formel (IV)

$$(CH_3)_3SiX \qquad (IV),$$

worin X für Halogen steht,

im Maße seiner Bildung laufend aus dem Reaktionsgemisch abdestilliert.

Es ist als ausgesprochen überraschend zu bezeichnen, daß Carbonsäureanhydride der Formel (I) nach dem erfindungsgemäßen Verfahren in hoher Ausbeute und Reinheit zugänglich sind, denn im Hinblick auf den bekannten Stand der Technik war zu erwarten, daß diese Reaktion überhaupt nicht stattfindet, da Trimethylsilylchlorid mit Carbonsäureanhydriden — in Umkehrung der erfindungsgemäßen Reaktion — unter Bildung von entsprechenden Carbonsäure-trimethylsilylestern und Carbonsäurechloriden reagiert (vgl. Organosilicon Compounds I, S. 62 (1965)).

Das erfindungsgemäße Verfahren besitzt eine Reihe von Vorteilen. So ist es nicht auf die Synthese weniger bestimmter Verbindungen beschränkt, sondern es läßt sich sehr breit anwenden und kann in einer Eintopfreaktion durchgeführt werden. Das Verfahren liefert Carbonsäureanhydride in praktisch quantitativer Ausbeute und ausgezeichneter Reinheit, frei von störenden oder umweltfeindlichen Nebenprodukten. Ein zusätzlicher entscheidender Vorteil des erfindungsgemäßen Verfahrens besteht darin, daß die Aufarbeitung keine Probleme bereitet; die Aufarbeitung erfolgt am einfachsten und zweckmäßigsten durch Destillation oder Umkristallisation.

Verwendet man Benzoesäuretrimethylsilylester und Benzoylchlorid als Ausgangsstoffe, so kann der Reaktionsverlauf durch das folgende Formelschema wiedergegeben werden:

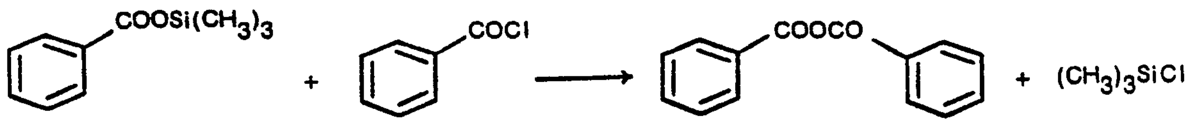

Die als Ausgangsstoffe verwendeten Carbonsäuretrimethylsilylester sind durch die Formel (II) und die Carbonsäurehalogenide durch die Formel (III) allgemein definiert und sind bekannt oder können nach bekannten Methoden synthetisiert werden (vgl. Organosilicon Compounds, I, S. 61 (1965); Organikum, 1967, S. 409).

In diesen Formeln stehen R bzw. R' vorzugsweise für geradkettiges oder verzweigtes Alkyl mit 1 bis 4 Kohlenstoffatomen, wobei jeder dieser Alkylreste substituiert sein kann durch Alkoxy mit 1 bis 4 Kohlenstoffatomen, Carbalkoxy mit 1 bis 4 Kohlenstoffatomen in der Alkoxygruppe, Nitro, Cyano und/oder Halogen (wie Fluor, Chlor, Brom oder Jod). Ferner stehen R bzw. R' vorzugsweise für gegebenenfalls durch Alkyl, Alkoxy oder Carbalkoxy mit jeweils bis zu 4 Kohlenstoffatomen, Nitro, Cyano und/oder Halogen (wie z.B. Fluor, Chlor und Brom) substituiertes Cycloalkyl mit 5 oder 6 Kohlenstoffatomen im Ringsystem. Weiterhin stehen R bzw. R' vorzugsweise für gegebenenfalls durch Alkyl, Alkoxy oder Carbalkoxy mit jeweils bis zu 4 Kohlenstoffatomen, Nitro und/oder Halogen (wie z.B. Fluor, Chlor und Brom) substituiertes Aryl, insbesondere Phenyl oder Naphthyl. Schließlich stehen R bzw. R' vorzugsweise für gegebenenfalls durch Alkyl, Alkoxy oder Carbalkoxy mit jeweils bis zu 4 Kohlenstoffatomen, Nitro, Cyano und/oder Halogen (wie z.B. Fluor, Chlor und Brom) substituierte 5- oder 6-gliedrige heterocyclische Reste, die 1 bis 3 Heteroatome wie Sauerstoff, Schwefel und/oder Stickstoff im Ring enthalten können und außerdem mit einem Benzolring anelliert sein können.

Als Beispiele für insbesondere in Frage kommende heterocyclische Reste seien genannt: Morpholinyl, Imidazolyl, Pyrazolyl, Pyrrolyl, Isoxazolyl, Piperidinyl, Oxazolyl, 1,2,4-Triazol-1-yl, 1,2,4-Triazol-4-yl, 1,2,3-Triazolyl, 1,2,4-Thiadiazol-2-yl, Benzimidazolyl und Furanyl.

Als bevorzugte Carbonsäuretrimethylsilylester der Formel (II) seien im einzelnen genannt: Essigsäuretrimethylsilylester, Propionsäuretrimethylsilylester, Buttersäuretrimethylsilylester, Pivalinsäuretrimethylsilylester, Hexancarbonsäuretrimethylsilylester, Dodecancarbonsäuretrimethylsilylester, Palmitinsäuretrimethylsilylester, Stearinsäuretrimethylsilylester, α-(2-[2,2-Dichlorovinyl]-3,3-dimethylcycloprop-1-yl)-α-carbonsäuretrimethylsilylester, Cyclopropancarbonsäuretrimethylsilylester, Methylcyclopropancarbonsäuretrimethylsilylester, Cyclobutancarbonsäuretrimethylsilylester, Methylcyclobutancarbonsäuretrimethylsilylester, Cyclopentancarbonsäuretrimethylsilylester, Cyclohexancarbonsäuretrimethylsilylester, Benzoesäuretrimethylsilylester, Chlorbenzoesäuretrimethylsilylester, Dichlorbenzoesäuretrimethylsilylester, Trifluormethylbenzoesäuretrimethylsilylester, Trifluoromethoxybenzoesäuretrimethylsilylester, Naphthalin-1-carbonsäuretrimethylsilylester, 1-Phenyl-5-pyrazolon-3-carbonsäuretrimethylsilylester, Terephthalsäuretrimethylsilylester, Isophthalsäuretrimethylsilylester.

Besonders bevorzugt sind Benzoesäure-trimethylsilylester und Pivalinsäure-trimethylsilylester.

Als bevorzugte Carbonsäurehalogenide gemäß Formel (III) seien im einzelnen genannt:

Acetylchlorid, Acetylbromid, Propionylchlorid, Propionylbromid, Buttersäurechlorid, Pivaloylchlorid, Hexancarbonsäurechlorid, Dodecancarbonsäurechlorid, Palmitinsäurechlorid, Stearinsäurechlorid, Cyclopropancarbonsäurechlorid, Methylcyclopropancarbonsäurechlorid, α-(2-[2,2-Dichlorvinyl]-3,3-dimethylcycloprop-1-yl]-α-carbonsäurechlorid, Cyclobutancarbonsäurechlorid, Methylcyclobutancarbonsäurechlorid, Cyclopentancarbonsäurechlorid, Cyclohexancarbonsäurechlorid, Benzoylchlorid, Benzoylfluorid, Chlorbenzoylchlorid, Dichlorobenzoylchlorid, Trifluoromethylbenzoylchlorid, Trifluormethylbenzoylfluorid, Trifluoromethoxybenzoylchlorid, Naphthalin-1-carbonsäurechlorid, 1-Phenyl-5-pyrazolon-3-carbonsäurechlorid, Terephthalsäuredichlorid, Isophthalsäuredichlorid. — Besonders bevorzugt sind Benzoylchlorid und Pivaloylchlorid. —

Als Carbonsäurehalogenide werden bevorzugt die Fluoride und besonders bevorzugt die Chloride verwendet.

Als Verdünnungsmittel, die bei der Durchführung des erfindungsgemäßen Verfahrens eingesetzt werden können, kommen alle inerten organischen Lösungsmittel, die weder mit den Carbonsäurehalogeniden der Formel (III) noch mit den Carbonsäuretrimethylsilylestern der Formel (II) und den Carbonsäureanhydriden der Formel (I) chemische Reaktionen eingehen, in Betracht. Solche Lösungsmittel sind beispielsweise die Xylole, wie o-Xylol, Chlorbenzol, o-Dichlorbenzol, die Trichlorbenzole, Nitrobenzol, Tetramethylensulfon. Im allgemeinen wird die erfindungsgemäße Umsetzung jedoch ohne Verdünnungsmittel durchgeführt.

Die Reaktionstemperatur kann in einem größeren Bereich variiert werden. Im allgemeinen arbeitet man bei Temperaturen zwischen 20°C und 250°C, vorzugsweise zwischen 50°C und 200°C.

Durch Zusatz von katalytischen Mengen Lewis-Säure kann die Umsetzung beschleunigt werden.

Als geeignete Lewissäure seien genannt: Zinkchlorid, Zinkcyanid, Kupfercyanid, Eisen-III-chlorid, Aluminiumchlorid, Bortrifluorid, Bortrifluorid-etherat, Triethylammoniumfluorid, Triethylammoniumfluorid x2 HF.

Die Aufarbeitung erfolgt nach beendeter Umsetzung üblicherweise durch Destillation oder Umkristallisation. Für viele weitere Umsetzungen der erfindungsgemäß synthetisierten Carbonsäureanhydride ist eine weitere Reinigung nicht notwendig.

Auch in der Gasphase kann das Gemisch aus Carbonsäuretrimethylsilylester und Carbonsäurehalogenid erfindungsgemäß umgesetzt werden.

In einer besonderen Verfahrensvariante läßt sich die erfindungsgemäße Umsetzung auch kontinuierlich gestalten.

Carbonsäureanhydride werden häufig verwendet als Ersatzchemikalien für Carbonsäurechloride mit einer vergleichbaren chemischen Reaktionsfähigkeit, aber ohne die bei der Verwendung von Säurechlorinden auftretende Korrosion zu verursachen. Auch die Verwendung von Carbonsäureanhydriden als Zwischenprodukte für Wirkstoffe des Pflanzenschutzes ist möglich: z.B. können die Carbonsäureanhydride (I) als Ausgangsstoffe zur Herstellung von Acylcyaniden (vgl. z.B. DE—OS 26 14 240, DE—OS 26 14 241, DE—OS 26 42 140, DE—OS 26 42 199) verwendet werden; die Acylcyanide können ihrerseits als Zwischenprodukte zur Synthese von 1,2,4-Triazin-5-onen, Verbindungen mit hervorragenden herbiziden Eigenschaften, eingesetzt werden (vgl. z.B. DE—OS 27 33 180, DE—OS 30 03 541, DE—OS 30 08 921, DE—OS 30 02 203, DE—OS 30 09 043).

So läßt sich z.B. Benzoesäureanhydrid nach einem bekannten Verfahren in den herbiziden Wirkstoff 3-Methyl-4-amino-6-phenyl-1,2,4-triazin-5-on (common name: Metamitron) umwandeln, indem man in einer ersten Stufe Benzoesäureanhydrid durch Umsetzung mit Alkalicyaniden oder wasserfreier Blausäure in Benzoylcyanid überführt, dieses in einer zweiten Stufe in Gegenwart von konzentrierter Salzsäure mit Ethanol umsetzt und den dabei entstehenden Phenylglyoxylsäureethylester in einer dritten Stufe mit Acetylhydrazin zur Reaktion bringt, wobei sich 1-Phenyl-glyoxylsäureethylester-2-acetylhydrazon bildet, das in einer vierten Stufe mit Hydrazinhydrat in Gegenwart von Pyridin in das oben erwähnte Endprodukt überführt wird (vgl. z.B. DE—OS 22 24 161, DE—OS 26 14 240, DE—OS 26 14 241).

Nach ebenfalls bekannten Verfahren kann Pivalinsäureanhydrid in den herbiziden Wirkstoff 3-Methylthio-4-amino-6-tert.-butyl-1,2,4-triazin-5-on (common name: Metribuzin) überführt werden (vgl. z.B. DE—OS 26 14 240 und DE—OS 26 14 241 in Verbindung z.B. mit DE—OS 30 09 043).

Die nachfolgenden Beispiele sollen zur weiteren Erläuterung der Erfindung dienen.

Beispiel 1 Benzoesäureanhydrid

In einem 250 ml-Dreihalskolben werden 97 g Benzoesäuretrimethylsilylester (0,5 Mol) auf 120—140°C erwärmt, dann werden 70,3 g Benzoylchlorid (0,5 Mol) innerhalb von 30 Minuten zugetropft. Das entstehende Trimethylsilylchlorid wird gleichzeitig abdestilliert. Der erhaltene Rückstand ist reines Benzoesäureanhydrid.

Ausbeute: 112 g (≙ 99 % d. Th.) Benzoesäureanhydrid;

Siedepunkt: 153—155°C bei 0,2 mbar.

Beispiel 2 Cyclohexancarbonsäureanhydrid

In einem 250 ml-Dreihalskolben werden 100 g Cyclohexancarbonsäure-trimethylsilylester (0,5 Mol)

und 73,3 g Cyclohexancarbonsäurechlorid (0,5 Mol) auf 140°C erwärmt und das entstehende Trimethylsilylchlorid wird innerhalb von 40 Minuten abdestilliert. Der Rückstand wird im Vakuum abdestilliert.

Ausbeute: 114 g (≙ 96 % d. Th.) Cyclohexancarbonsäureanhydrid;
Siedepunkt: 128—131°C bei 0,2 mbar

Beispiel 3 4,4'-Dichloro-benzoesäureanhydrid

In einem 250 ml-Dreihalskolben mit Rührer, Thermometer und Destillationsbrücken werden 114,3 g 4-Chlorobenzoesäuretrimethylsilylester (0,5 Mol) und 87,8 g 4-Chlorobenzoylchlorid (0,5 Mol) gemischt und auf 150°C erwärmt. Es erfolgt nur eine geringe Abspaltung von Trimethylsilylchlorid. Nach Zugabe von 0,1 g Zinkchlorid muß bei dieser Temperatur das Trimethylsilylchlorid zügig abdestilliert werden. Nach beendeter Umsetzung wird der Rückstand aus Chlorobenzol umkristallisiert.

Ausbeute: 139 g (≙ 94 % d. Th.) 4,4'-Dichlorobenzoesäureanhydrid;
Schmelzpunkt: 195°C.

Analog zu den Beispielen 1—3 können auch die in der folgenden Tabelle 1 aufgeführten Carbonsäureanhydride (I) hergestellt werden:

Tabelle 1

| BSp. Nr. | Carbonsäureanhydrid (I) | Ausbeute | Siedepunkt (Kp); Schmelzpunkt (Fp.) |
|---|---|---|---|
| 4 | $(CH_3—CO—)_2O$ | 85 % | Kp.: 138—140°C |
| 5 | $(CH_3—CH_2—CO—)_2O$ | 96 % | Kp.: 168—169°C<br>67,5°C/24 mbar |
| 6 | $[(CH_3)_3C—CO—]_2O$ | 95 % | Kp.: 83—85°C/<br>20 mbar |
| 7 | 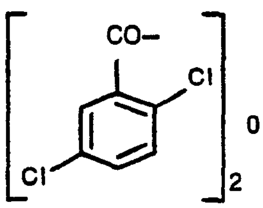 | 87 % | Kp.: 208—211°C/<br>0,266 mbar<br>Fp.: 107—109°C |
| 8 | 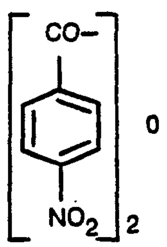 | 86 % | Fp.: 186—190°C |
| 9 | 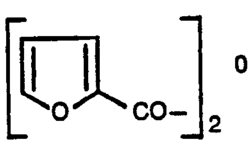 | 84 % | Kp.: 153—168°C/<br>0,4 mbar<br>Fp.: 70—72°C<br>(GC: 99 % Reinheit) |
| 10 | 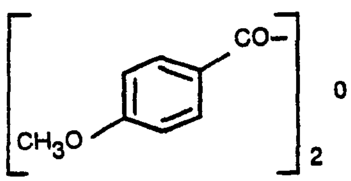 | 95 % | Fp.: 97—99°C |
| 11 | 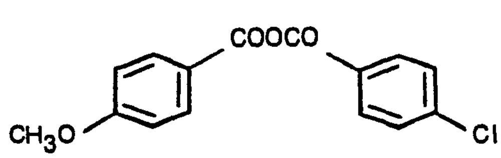 | 93 % | Fp.: 167—169°C<br>(aus Chlorbenzol) |

**Patentansprüche**

1. Verfahren zur Herstellung von Carbonsäureanhydriden der allgemeinen Formel (I)

$$R—CO—O—CO—R' \qquad (I)$$

in welcher

R und R' gleich oder verschieden sein können und für gegebenenfalls substituiertes Alkyl mit 1 bis 18 Kohlenstoffatomen, gegebenenfalls substituiertes Cycloalkyl mit 3 bis 12 Kohlenstoffatomen, gegebenenfalls substituiertes Aryl oder für einen gegebenenfalls substituierten 5- oder 6-gliedrigen heterocyclischen Rest stehen, der zusätzlich noch mit einem Benzolring annelliert sein kann,

dadurch gekennzeichnet, daß man Carbonsäuretrimethylsilylester der allgemeinen Formel (II)

$$R—CO—OSi(CH_3)_3 \qquad (II)$$

in welcher

R die oben angegebene Bedeutung hat,

mit Carbonsäurehalogeniden der allgemeinen Formel (III)

$$R'—CO—X \qquad (III)$$

in welcher

R' die oben angegebene Bedeutung hat, und

X für Halogen steht,

gegebenenfalls in Gegenwart eines Katalysators und gegenbenenfalls in Gegenwart eines Verdünnungsmittels bei Temperaturen zwischen 20 und 250°C umsetzt und das im Reaktionsverlauf in äquivalenter Menge entstehende Trimethylsilylhalogenid der Formel (IV)

$$(CH_3)_3SiX \qquad (IV)$$

worin X für Halogen steht,

im Maße seiner Bildung laufend aus dem Reaktions-gemisch abdestilliert.

2. Verfahren nach Anspruch 1, dadurch gekennzeichnet, daß man die Umsetzung bei Temperaturen zwischen 50 und 200°C durchführt.

3. Verfahren nach Anspruch 1, dadurch gekennzeichnet, daß man als Carbonsäurehalogenide (III) die Fluoride und insbesondere die Chloride einsetzt.

4. Verfahren nach Anspruch 1, dadurch gekennzeichnet, daß man die Umsetzung in Gegenwart einer Lewis-Säure als Katalysator durchführt.

5. Verfahren nach Anspruch 1, dadurch gekennzeichnet, daß als Carbonsäure-trimethylsilylester (II) Benzoesäuretrimethylsilylester und als Carbonsäurehalogenid (III) Benzoylchlorid eingesetzt werden.

6. Verfahren nach Anspruch 1, dadruch gekennzeichnet, daß als Carbonsäure-trimethylsilylester (II) Pivalinsäure-trimethylsilylester und als Carbonsäurehalogenid (III) Pivaloylchlorid eingesetzt werden.

**Revendications**

1. procédé de production d'anhydrides d'acides carboxyliques de formule générale (I)

$$R—CO—O—CO—R' \qquad (I)$$

dans laquelle

R et R' peuvent être égaux ou différents et représentent un groupe alkyle éventuellement substitué ayant 1 à 18 atomes de carbone, un groupe cycloalkyle éventuellement substitué ayant 3 à 12 atomes de carbone, un groupe aryle éventuellement substitué ou un reste hétérocyclique pentagonal our hexagonal éventuellement substitué, qui peut en outre être encore condensé avec un noyau benzénique,

caractérisé en ce qu'on fait réagir des esters de triméthylsilyle d'acides carboxyliques de formule générale (II)

$$R—CO—OSi(CH_3)_3 \qquad (II)$$

dans laquelle

R a la définition indiquée ci-dessus,

avec des halogénures d'acides carboxyliques de formule générale (III)

$$R'—CO—X \qquad (III)$$

dans laquelle

R' a la définition indiquée ci-dessus, et

X désigne un halogène,

éventuellement en présence d'un catalyseur et en la présence éventuelle d'un diluant à des températures

de 20 à 250°C, et on chasse continuellement du mélange réactionnel, par distillation, à mesure de sa formation, l'halogénure de triméthylsilyle produit en quantité équivalente au cours de la réaction, de formule (IV)

$$(CH_3)_3SiX \qquad (IV)$$

dans laquelle X désigne un halogène.

2. Procédé suivant la revendication 1, caractérisé en ce qu'on conduit la réaction à des températures comprises entre 50 et 200°C.

3. Procédé suivant la revendication 1, caractérisé en ce qu'on utilise comme halogénures d'acides carboxyliques (III) les fluorures et notamment les chlorures.

4. Procédé suivant la revendication 1, caractérisé en ce qu'on conduit la réaction en présence d'un acide de Lewis comme catalyseur.

5. Procédé suivant la revendication 1, caractérisé en ce qu'on utilise comme ester de triméthylsilyle d'acide carboxylique (II) l'ester de triméthylsilyle de l'acide benzoïque et, comme halogénure d'acide carboxylique (III), le chlorure de benzoyle.

6. Procédé suivant la revendication 1, caractérisé en ce qu'on utilise comme ester de triméthylsilyle d'acide carboxylique (II), l'ester de triméthylsilyle de l'acide pivalique et comme halogénure d'acide carboxylique (III), le chlorure de pivaloyle.

**Claims**

1. Process for the preparation of carboxylic acid anhydrides of the general formula (I)

$$R—CO—O—CO—R' \qquad (I)$$

in which

R and R' can be identical or different and represent optionally substituted alkyl with 1 to 18 carbon atoms, optionally substituted cycloalkyl with 3 to 12 carbon atoms, optionally substituted aryl or an optionally substituted 5- or 6-membered heterocyclic radical which can additionally be fused to a benzene ring,

characterised in that carboxylic acid trimethylsilyl esters of the general formula (II)

$$R—CO—OSi(CH_3)_3 \qquad (II)$$

in which

R has the meaning given above,

are reacted with carboxylic acid halides of the general formula (III)

$$R'—CO—X \qquad (III)$$

in which

R' has the meaning given above and

X represents halogen,

if appropriate in the presence of a catalyst and if appropriate in the presence of a diluent at temperatures between 20 and 250°C and the trimethylsilyl halide of the formula (IV)

$$(CH_3)_3SiX \qquad (IV)$$

wherein

X represents halogen,

which is formed in the course of the reaction in an equivalent quantity is continuously distilled off from the reaction mixture at the rate at which it forms.

2. Process according to Claim 1, characterised in that the reaction is carried out at temperatures between 50 and 200°C.

3. Process according to Claim 1, characterised in that the carboxylic acid halides (III) used are the fluorides and in particular the chlorides.

4. Process according to Claim 1, characterised in that the reaction is carried out in the presence of a Lewis acid as the catalyst.

5. Process according to Claim 1, characterised in that benzoic acid trimethylsilyl ester is used as the carboxylic acid trimethylsilyl ester (II) and benzoyl chloride is used as the carboxylic acid halide (III).

6. Process according to Claim 1, characterised in that pivalic acid trimethylsilyl ester is used as the carboxylic acid trimethylsilyl ester (II) and pivaloyl chloride is used as the carboxylic acid halide (III).